# EUROPEAN PATENT SPECIFICATION

(11) **EP 2 785 811 B1**
(45) Date of publication and mention of the grant of the patent: **22.07.2020**
(21) Application number: 12854254.5
(22) Date of filing: 03.12.2012
(51) Int. Cl.: C09K 3/32, B01J 20/10, B09C 1/10, A62D 3/02, C12N 11/14, C02F 3/34

(54) **COMPOSITION FOR SOAKING UP AND BIODEGRADATION OF OIL AND ORGANIC CHEMICALS**
ZUSAMMENSETZUNG FÜR AUFSAUGEN UND BIOLOGISCHEN ABBAU VON ÖL UND ORGANISCHEN CHEMIKALIEN
COMPOSITION DESTINÉE À ABSORBER ET À BIODÉGRADER DES PRODUITS PÉTROLIERS ET DES PRODUITS CHIMIQUES ORGANIQUES

(30) Priority: 02.12.2011 FI 20116219
(43) Date of publication of application: 08.10.2014
(73) Proprietor: Algol Chemicals Oy, 02610 Espoo (FI)
(72) Inventor: POHJOLA, Pekka, 17800 Kuhmoinen (FI)
(74) Representative: Berggren Oy, Helsinki & Oulu
(86) International application number: PCT/FI2012/051198
(87) International publication number: WO 2013/079805

(56) References cited:
- WO-A1-01/10549
- WO-A1-01/32588
- WO-A2-00/13787
- GB-A- 1 461 577
- US-A- 5 700 558
- US-A1- 2011 082 040

## Description

The object of the invention is a composition for soaking up and biodegradation of oil products and other organic chemicals. The invention also relates to a method for manufacturing and using such a composition.

### Prior art

Oil is one the most important raw materials in industrialized society, but being a fossil material, it exists only in particular areas. It is chartered for refinement from oil-drilling areas to all over the world. Oil industry, storage and logistics form a global network that is vulnerable to risks due to the nature of oil products. (Raw Crude oil) Petroleum and oil products are harmful to the environment and oil pollution damage always requires oil destruction operations. Small oil pollution damages occur daily, but disaster-scale catastrophes are also common. Under arctic conditions, a large catastrophe can prove disastrous. Many sea birds, mammals, fishes, plankton and bottom organisms are in danger. At the beginning oil makes a floating layer on water and usual technical destruction ways are collecting, absorption or burning. Also offshore, chemical detergents have been used, that degrade oil to smaller hydrocarbon molecules and that enable mixture of oil with water. This, however, makes oil toxic to micro-organisms and can cause unforeseeable inconvenience.

Oil is a natural product and it biodegrades under advantageous conditions. Many microbe species, that are common in nature, are able to use oil as a carbon source. Small molecule fractions, which are the most toxic, degrade the fastest, but bigger polyaromatic fractions demand more time. When oil flows into water there starts many physical reactions for example: emulsification, mixing, spreading, evaporation, dissolving and sedimentation. The biodegradation demands water, oxygen and nutrients - mainly nitrogen and phosphorus. The temperature is also an active factor. For aerobic degradation it is important that the microbial respiration is possible and nutrients and water are present just in the functional phase. Because of disadvantages in these circumstances oil degrades usually slowly in nature.

The conventional methods that are used for removing oil from water involve spreading adsorbent materials such as clay or silica particles often coated with an oleophilic agent onto oil. The adsorbents are preferably naturally occurring clays, such as kaolinic clay. Oleophilic factor can be obtained for instance by vegetable-oil coating. These materials are removed from the water surface mechanically. The adsorbents are normally added so much that adsorbent hydrocarbon mixture is a dry powder. Such materials that sink after adsorbing leave masses of oil on the bottom of water. The sunken mass is mobile and could be released and be harmful for fishes and bottom animals. It has also used nutrients and special freeze dried microorganisms packed into mineral particles as aim to convert oil to degradation products. So, we can suppose that oil can be left in nature after the treatment and is digested by micro-organisms.

WO 00/13787 disclosed an oil sorbent composition provided as an admixture to create and maintain a separation between different components.

WO 01/10549 discloses a sorbent used to purify water and soil contaminated by oil products. Said sorbent contains an alumino silicate-based carrier with immobilized microorganisms in the presence of phosphor- and nitrogen-containing salts.

US 2011/082040 discloses compositions and methods of delivering substances in a dry mode. E.g. essential oils or microorganisms can be loaded throughout the pores of the precipitated silica granules.

US 5 700 558 discloses a biodegradable absorbent material for oil or other hydrocarbon liquid spills. The material consists of a mixture of granulated foam, microbial nutrient, de-dust agent and ground cellulosic material.

GB 1 461 577 discloses a method of reducing oil pollution on the surface of water and a particle having a porous core having a film of paraffin on the surface region usable in said method.

Liquid hydrocarbons have a low surface tension so they spread efficiently in water, on solid surfaces, in soil, and in snow and ice in arctic circumstances. Wide spread oils are difficult to remove and there often are large quantities of contaminated masses. Oil with water can, additionally, make emulsions and separation of (where) microscopic disperse oil balls is technically difficult and expensive.

### Summary of the invention

The object of this invention is a composition for soaking up and biodegradation of oil products and other organic chemicals that accelerates biodegradation very efficiently. The object of this invention is also a method for manufacturing and using said composition in different processes and apparatus.

To reach this object, the invention is characterised by features which are presented in the independent claims. The other claims present some advantageous embodiments of the invention.

A composition having at least a mineral carrier having pores and having oleofobic surface, and comprising nitrogen nutrients and phosphorus nutrients is disclosed.

The aim of the invention is to get nutrients and other vital substances in water insoluble form into contact with oil molecules so that the microbe action is effective. The invention is based on the finding that the biodegradation is effective on the surface of mineral particles when nutrients are available. The surface of the composition works as catalytic carrier where oil concentration is not too toxic and microbes can utilize water, nutrients and oil carbon sufficiently. The composition has additionally surface and colloid chemical influences that reduce the oil surface in water and make oil colloidal state like gel. So the absorption and gathering of oil are easier.

This invention comprises compositions and methods for manufacturing, removing and digesting oils and other hydrocarbons, oxygenated and chlorinated hydrocarbons, singly and in combinations, and particularly those found in naturally occurring substances known as crude oils, gas oils and petroleum.

This disclosure comprises organic fibre absorbents used in combination with composition digesting and removing oil and similar hydrocarbons and/or organic chemicals.

The composition disclosed here can be used in different ways and in different apparatus. The process/apparatus type can be e.g. liquid and/or in solid phase; patch or continuous process. It can be realized in fluids or solid phases in surface, in mixed or unmixed process/apparatus depending on embodiment and environmental conditions and extensions. Also used composition affects what kind of process/apparatus is advantageous to use.

The term nutrients as used herein comprises those inorganic substances which the microorganisms require for growth and activity. In table 1 are common elements that microbes usually need. That set is not exclusive, however, of vital importance are carbon and water. Carbon is a structural and energetic factor and water maintain life. Many microorganisms can digest carbon from hydrocarbons if they can supply water and nutrients simultaneously. Supply of water often is a limiting factor for biodegradation in hydrophobic materials.

The hydrocarbon consuming or converting microorganisms include bacteria, molds, yeast, fungi and actinomyces that often already are present, in natural oils, in carrier composition or in environment; in water, earth or air.

**Table 1.**

| **Group I** | **Group II** | **Group III** | **Group IV** |
|---|---|---|---|
| Carbon | Phosphorus | Potassium | Iron |
| Nitrogen | Sulphur | Sodium | Manganese |
| Oxygen | | Magnesium | Copper |
| (Water) | | Calsium | Cobalt |
| | | | Molybdenum |
| | | | Zinc |
| | | | Vanadium |

The mineral carrier material include clays such as kaoline and calcium bentonite, zeolites and other microporous silica alumina materials and precipitated silica granules having porous structure, a particle size of 10 - 10 000 micrometers, the pore sizes nanometers so that nutrients can be loaded into pores. The vegetable oil is selected palm oil, sunflower oil, rapeseed oil, soya oil, linseed oil, olive oil, coconut oil, a constituent or derivative of said oils and mixtures thereof. Clay or silica can also be a by or waste product from glass - , mining-, or chemical industry. An advantageous solution is to use filter clay from biofuel manufacturing process where filter cages may include 10 - 40% remainder bio oils and fats.

The oleophilic organic fibre absorbent is selected cellulose like cellu-cotton, wood cellulose, recycled paper cellulose, linen wool, cotton grass, cutter wood chips, sawdust, straw or other natural environment friendly material.

A mineral carrier coated by oleophilic vegetable oil has negative charge. By metal cations like ferrous ions the carrier is converted to an oleophobic material Additionally treated with alkali carbonates which are saponifying agents with vegetable oils they cause a surface tension factor into carrier mineral when added into oily water. The nutrients like ammonium and potassium needed in the biodegradation can be placed into pores of the silica. A vital element, phosphorus can be loaded into carrier as water insoluble form with iron. When added into crude oil or other similar hydrocarbon on the surface of water or in other wet environment the oil is accumulated as a gel like form. The oily area on the surface of water after the treatment is a fraction part of original contaminated. The accumulation process is quick and retracting of an oily area of one square metre in the laboratory test took about ten seconds. The gel like oil in small scale is easy to remove by hand, by filter or in greater accidents by absorbing wool or mats or by brushing machines. In soil, snow or in ice the spreading of oil is stopped after addition of composition. The accident is then in control and oil gel is easier to remove.

It is evident that a mineral particle acts as an active, catalytic centre in the composting process (EP 1252124). When spreaded on oil layer in composition disclosed here the microorganisms are in safe in otherwise toxic environment. They can use vital nutrients loaded into particles and carbon from oil substrate. The silica structure and surface tension factors serve circumstances where microorganisms can utilize water also in hydrophobic matter. The cleaning of environment can further be improved if composition is used in combination with oleophilic absorption material. For instance the crude oil after treated by the powder composition climbs quickly along linen fibres.. The oil in fibres is consumed efficiently by microbes assisted by nutrients in the silicaThe specific uses of this composition are manifold. Small private oil emissions from water or solid surfaces are easily cleaned by composition and remnants are biodegradable. In bigger oil damages for instance on the sea, harbours and shorelines the composition can be used with oil booms after the oil is arrested. The composition may be dispersed from boats, aircraft or manually carried containers. Quick spreading of oleophobic powder on shoreline when oil threats may prevent oil coming ashore. The oil gel can be gathered by brushing machines or even by nets if absorption wools are used.

The composition serves a good solution into cleaning of oily waste waters. Wide spread oil or emulsions are difficult to filter from water. If the oil or other similar hydrocarbons are converted to gels by the composition disclosed here the separation of oil is managed by coarse filter or by column technology.

Biologic treatment is wide used and cheap technique for contaminated soils but it often fails. This catalytic composition brings new possibilities into biodegradation. There are nutrients already in the structure and microorganisms grow also in high toxic circumstances.

The composition is functional and active also in arctic circumstances. It makes the oil destruction easier in ice and snow and biodegradation is possible also in cold circumstances if only water stays in liquid form.

The manufacturing of the composition is cheap because of using by and waste products. Silicate cages from one bio oil refinery come thousands of tons and there are vegetable oils present already. The metal sulphates are cheap and as absorption material can be used for instance recycled paper cellulose. Nitrogen and phosphorus sources are optional. Mixers are the only apparatus needed in manufacturing..

The mineral carrier forms the base of the composition, which acts as a binder of nutrients for microbes and also has surface catalytic effects, ion exchange effects and molecular sieve effects. Nitrogen and phosphorus nutrients, which have bound to the mineral particles, in direct contact with oil and similar organic hydrocarbons and organic chemicals that act as a carbon source, enable the vital functions of natural microbes and adaptation to the hydrocarbon-rich operating environment. Nitrogen and phosphorus are essential elements for microbes, and microbes use them to proliferate and degrade hydrocarbons in order to acquire carbon, which is essential for them to function. Amount of ammonium-nitrogen can e.g. be 0.5 to 5 %,(w/w), such as 1.5 to 2.5 % (w/w). Amount of phosphorous can e.g. be 0.005 to 0.5 % (w/w), such as 0.1 to 0.3 5 (w/w).

According to the disclosure there is also be a native microbe strain in the composition. This adds biodegradation speed in certain applications.

According to the disclosure there is vegetable oil or fat in the composition and carbonate compound(s), which together makes oleofobic surfaces on the mineral particles. Preferable there is also iron. Those particles change the zeta-potential and surface tension so that area of the oil in water is reduced making oil state like gel. Amount of vegetable oil and fat can e.g. be 0.2 - 50%(w/w), such as 1 to 20 % %. The vegetable oil is for example such as palm oil, sunflower oil, rapeseed oil, soya oil linseed oil, olive oil, coconut oil, a constituent or derivative of said oils and mixtures thereof. Amount of carbonate compound can e.g. be 0.2 -2 % (w/w), such as 0.25 to 1.0 %. According to the disclosure, the mineral carrier particles is a surface catalytic material, such as a natural or synthetic silicate mineral. The mineral carrier is a natural or synthetic fine silicate, for example, such as kaoline and calcium bentonite, zeolites and other microporous silica alumina materials and precipitated silica granules having porous structure, a particle size of 10 - 10 000 micrometers. The mineral carrier may originate directly from nature or it may be a silicate precipitate that is produced as a by-product of industrial activity, such as glass-, mining- or chemical industry. The microstructure of particular minerals comprises passages cages and pores sizes of nanometers that arrest nutrients and further enhance catalytic degradation. Amount of mineral carrier in composition can e.g. be 50 to 90 % (w/w), such as 75 to 85 % (w/w).

As disclosed here, the composition further comprises iron and/or manganese. Iron preferably binds phosphorus to the composition, for example. Preferable iron and manganese are both present in said composition. These components interact so that the composition works especially well in water element. Amount of iron can e.g. be 0.2 to 6 (w/w), such as 1.0 to 4(w/w). Amount of manganese can e.g. 0.05 to 3 % (w/w), such as 0,2 to 1% (w/w). Nitrogen is bound as insoluble ammonium ion in porous mineral structure. Iron precipitation and mineral particles make phosphorus insoluble.

As disclosed here, the composition includes copper, cobalt and molybdenum as micronutrients. These are important micronutrients for microbes and help to accelerate the action of microbes. Ingredients, that promote cell respiration and redox-activity of microbes, enhance and diversify the action and proliferation of microbes. As disclosed here, the composition further comprises an ingredient that absorbs oil and similar hydrocarbons and/or organic chemicals, such as cellulose cotton, wood cellulose, recycled paper cellulose, linen wool, cotton grass, cutter wood chips, sawdust, straw or other biodegradable vegetable fibre. The solution essentially improves the collecting oils and chemicals and aerobic biodegradation goes effectively in fibres with nutrients in mineral particles.

Depending on an application, the mineral carrier can be lighter than water, heavier than water or equal in weight with water. Nutrients packed into the floating mineral particles, for example, can start the degradation process even on the surface of water. Floating powder in combination with the absorbing material, such as cellulose fibre, also acts as an efficient oil collector.

There are many applications for the product that absorb and enhance the biodegradation of oil and oil products ranging from small pollution accidents to major environmental accidents. The powder can be spread as such on the oil or it can be used in combination with the absorber. The composition may be dispersed manually from containers or on the sea from aircraft or from boats. It has been demonstrated experimentally, that about 50 grams of cellulose fibre composition, into which the reactive powder has been bound, can absorb about one kilogramme of raw oil. Natural microbes capable of degrading oil produce a rapidly growing microbe-culture on the oil. Finally, the biodegradable oil absorbing fibre material also biodegrades.

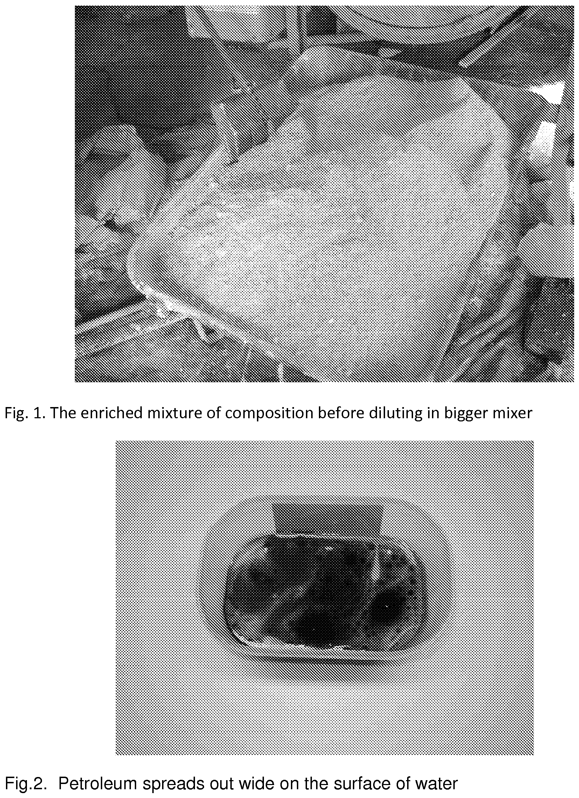

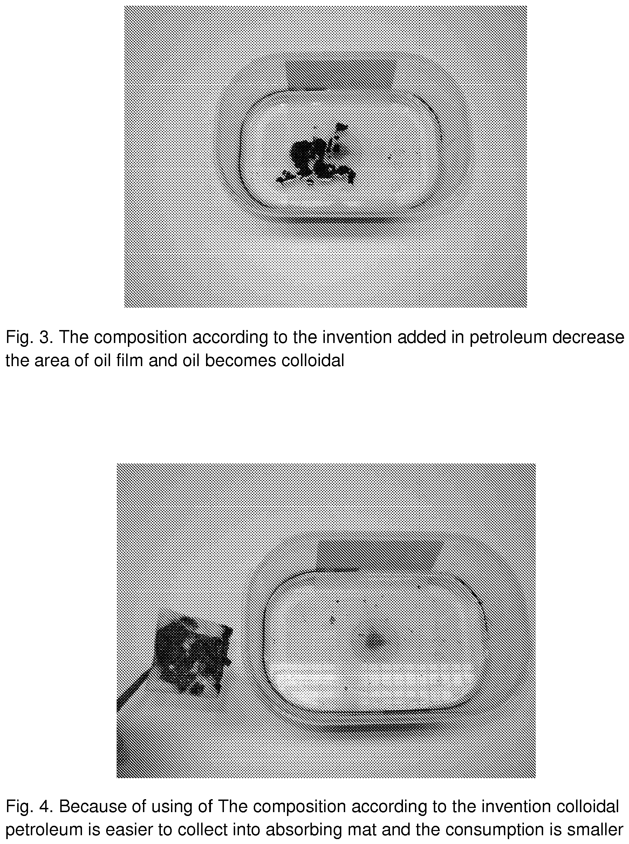

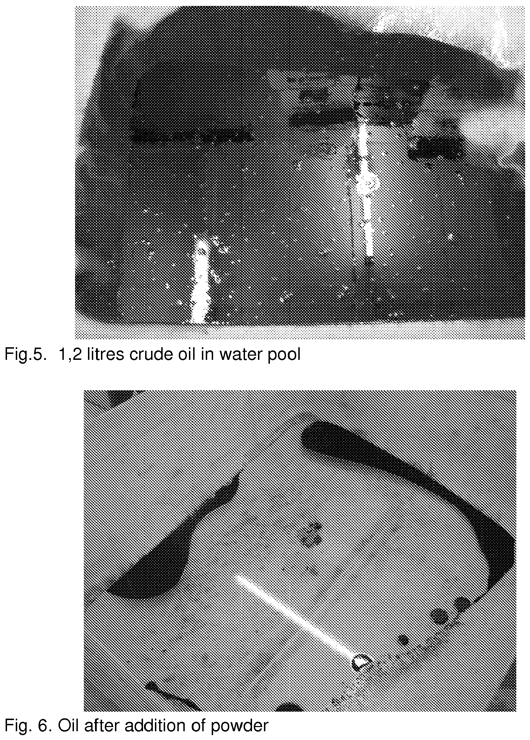

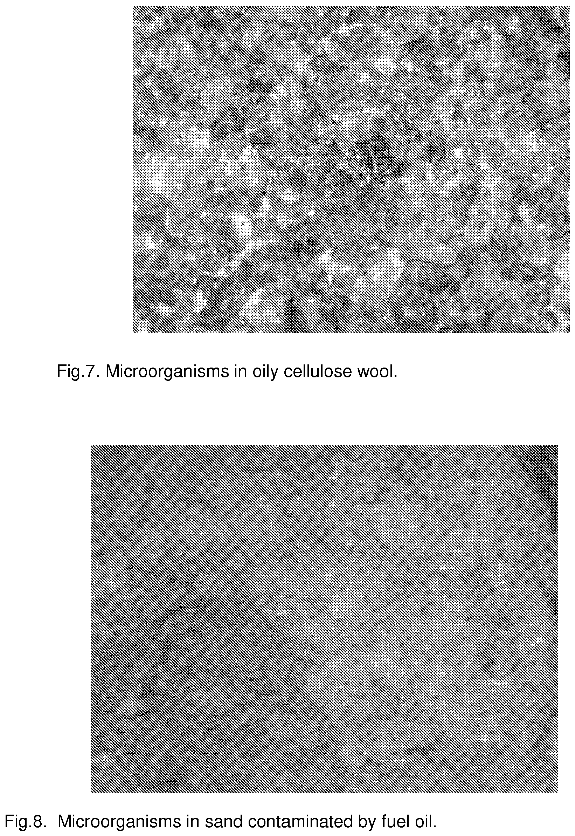

The experiments have been illustrated in figures 1 and 2. In fig. 1 it is shown crude oil in water and the composition of the invention. In fig. 2 it is shown reduced surface because of adding the composition. In fig. 3. it is demonstrated how components added in certain order into mineral powder produce functional composition for oil destruction. Fig. 4 shows how petroleum spreads out wide on the surface of water depending on surface tension. Fig. 5 shows how the composition according to the invention added in petroleum decrease the area of oil film and oil becomes colloidal. Fig. 6 shows how the use of the composition according to the invention colloidal petroleum is easier to collect into absorbing mat and the consumption is smaller. Fig. 7 shows that the composition according to the invention added on the surface of petroleum in water biodegrades oil by many species of natural micro organisms in some months without sediments.

It was found that when adding 10g composition to water having 1kg crude oil on it, oil is rapidly retracting and thickening. It is thereafter easy to absorb and collect. Need of absorption material is reduced to 1/10 by this procedure. It is also easier to gather formed gel material compared to crude oil.

The composition disclosed here can be used in oil damages for soaking oil and also for filtration of oily water. Formed waste can be composted. Contaminated soil and other mass can be composted by this new composition, too.

The appended examples and figures describe some applications of the invention.

Appended Figures:
Fig. 1. The enriched mixture of composition before diluting in bigger mixer
Fig.2. Petroleum spreads out wide on the surface of water
Fig. 3. The composition according to the invention added in petroleum decrease the area of oil film and oil becomes colloidal
Fig. 4. Because of using of The composition according to the invention colloidal petroleum is easier to collect into absorbing mat and the consumption is smaller
Fig.5. 1,2 litres crude oil in water pool
Fig. 6. Oil after addition of powder
Fig.7. Microorganisms in oily cellulose wool.
Fig.8. Microorganisms in sand contaminated by fuel oil.
Fig. 9. Accumulated crude oil on the surface of water treated by composition according to the invention with linen fiber
Fig. 10. Oil flocculates occupied by microorganisms activated by composition according to the invention on the surface of water
Fig. 11. Insoluble microbe production (humus) and blackened remains of diesel oil from the biodegradation test by oil eating powder on the surface of water
Fig. 12. Biodegradation of different oils on the surface of water by the composition according to the invention.
Fig. 13. Crude oil arrested in small area in snow by composition
Fig. 14. Crude oil removed from snow by absorption wool
Fig. 15. Microorganisms in the vessel where the composition has been added into crude oil in snow.

### Example 1.

The test portion of the composition according to the invention was manufactured as follows. 60 kg of dry silicate cage filter material from bio oil refinery (organic substances like oil remnants 20 - 40%) was transferred into a cement mixer. Silicate was mixed first with 50 kg of ammonium sulphate followed by 35 kg of ferro sulphate and 2 kg of manganese sulphate. 10 kg of potassium carbonate caused a little ammonia smell when reacted with former substances. The mass was let stand over night until it was pale brown. Then there was added an additional portion 50 kg of di-ammonium hydrogen phosphate and trace element mixture. After the thorough mixing the enriched mixture (Fig. 1) was removed into a big mixer where it was diluted with 800 kg of same silicate as started with.

### Example 2.

1 ml of Russian crude oil was added into 200 ml of water. Oil makes a thin film on the surface of water that is difficult to remove (Fig. 2). A couple of milligrams of the composition according to the invention cause the accumulation of oil film (Fig.3.) The oil flocculate is easy to remove by oil absorbing material such as straw, cellulose, linen or oil absorbing mat (Fig. 4)

### Example 3.

Into 50 ml syringe was installed 0,2 grams of composition according to the invention mixed with 0,5 grams of linen wool. Another syringe was equipped with pure linen wool. 150 ml portions of oily water (3% diesel oil) was let run through both syringes. The phenomenon that can be compared with flocculation caused by the powder composition arrest efficiently oil into linen. The oil analyses of linen filter pointed out that 99,8 % of diesel oil was arrested into linen wool and water was clean. If there was only linen wool filter without powder only 42 % of oil was arrested into linen wool and oily water did not clean.

### Example 4.

1. 1200 grams of Russian crude oil was added into the pool, surface area of 100 x 100 cm where oil was spread as an all coating film (Fig.5). By 5 grams of the powder according to the invention the oil film was reduced to 5 percents from original area (Fig. 6). After that treatment it was easy to absorb the oil by small amount of mixture of powder and cellulose wool (20g + 180g). The wet oily mass of 2 100 grams was removed into small *Compomate* indoor composter (pat FI 113165).
2. 800 grams of fuel oil was correspondingly added into the basin and collected by same method by 60 g of cellulose wool mixture into the composter the total mass being 1550 grams.

Both oily masses were composted aerobically six months and oil contents were analyzed by hexane extracting method.

In spite of very high oil contents visibly microorganism compositions into both composters were formed in the couple of days (Fig. 7).The temperature during first three months was somewhat low in the composting room(5 - 10°C) but later 15 -20 °C.

The total mass of the compost 1. was reduced 34% in three months and 42 %in six months . The reductions in composter 2. were correspondingly 45 and 61 %. The loss of weigh is due to the evaporating of water, carbon dioxide and metabolic products. Partly the oil is changed to the black insoluble biomass which is cumulated into the solid phase.

According to the oil analyses the crude oil content by the composition according to the invention during three months was reduced 53% and in six months 72% and fuel oil content in three months 72% and in six months 84 %.

### Example 5.

1. 180 grams of crude oil was added into 5 kg of sandy soil. This was mixed with mixture of cellulose wool and the powder according to the invention (50g +150 g). The oily soil was removed into Compomate composter.
2. 100 grams of fuel oil was mixed with 5 kg of sandy soil then added mixture of 50 g powder and 100 grams of linen wool and removed into composter.

Visible microorganisms were appeared into composters in three days (Fig. 8)) and oily smell was nearly disappeared. Oil analyses after three months indicated that crude oil was left 180 mg/kg and fuel oil 36 mg/kg of dry matter. The losses of total weigh were 9,8 and 13,2%.

### Example 6.

The composition according to the invention was tested with soil contaminated by KY-5 chlorophenols. Originally there were over 2 500 milligrams per kilo chlorophenols in the soil from an old sawmill. 5 kilos of soil was treated with 100g of the powder according to the invention. The contaminated soil then was composted in the Compomate composter. The analyses indicated that the total chlorophenol concentration was under 150 mg/kg after three months.

### Example 7.

The degradation of oils on the surface of water by the composition according to the invention was tested in five different pools. The oils were: The Russian crude oil, fuel oil (diesel) and waste lubricating oil from diesel engine. The degradation was tested by pure powder product and with two absorbents, powder in cellulose wool and linen wool. The accumulation of oil films was produced by small portions of powder like in the example 2. Then additional powder or mixture of wool and powder were added on the oil flocculants. The portions of oil were about 120 grams and powder or/and wool was used about 5 grams.

The degradation layers were carefully collected after three months and oil remaining was determined by hexane extraction.

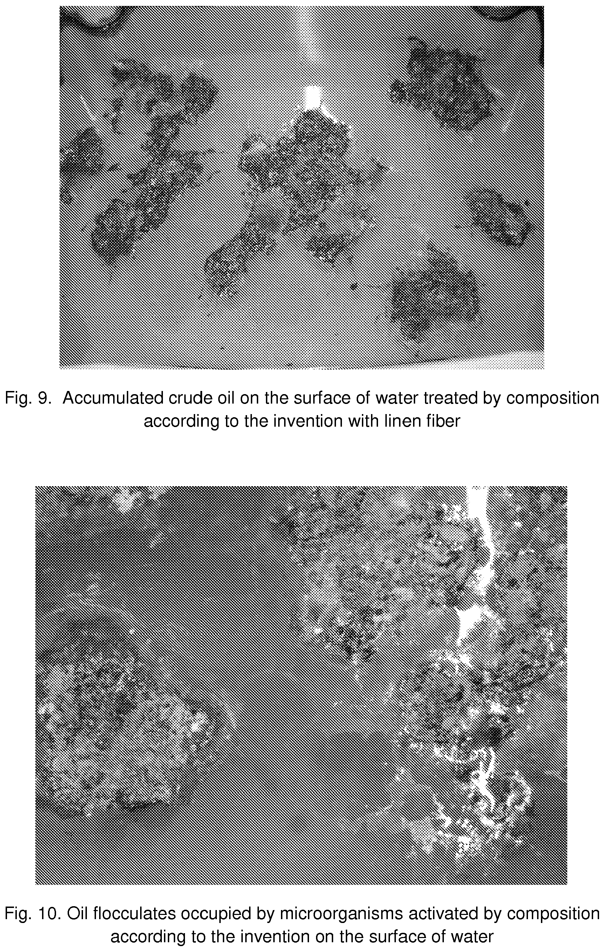

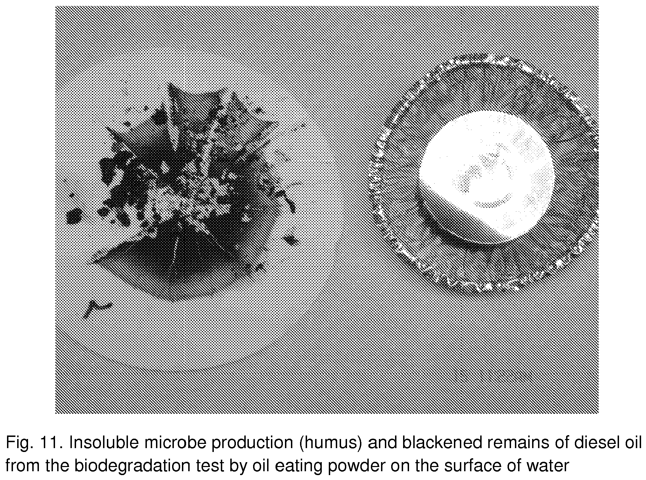

The surface areas of oil layers on water were sufficiently small after addition of powder (Fig. 9). The microbe population was very dense all the test time (Fig. 10). The oil analyses indicated that at its best over 90 per cent of the oil was removed from the surface during the test. Simultaneously black insoluble metabolic products (humus) were developed (Fig. 11). All the material was floating, however, and no sunken mass was detected .

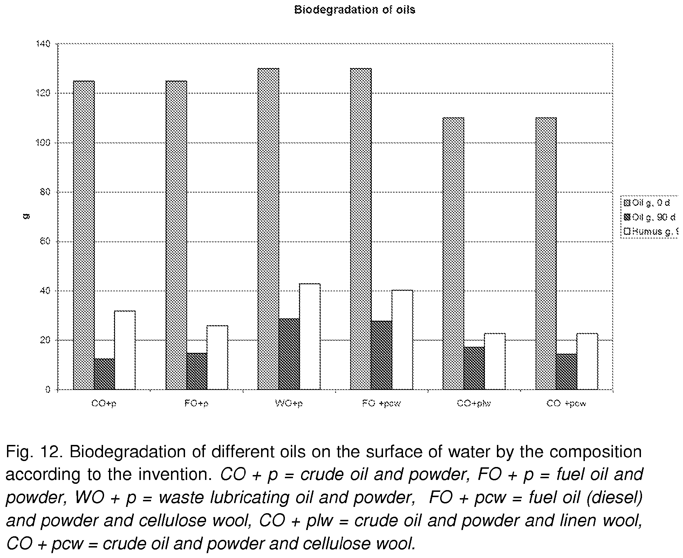

In Fig.12 are presented the remains of oils and amounts of humus developed during the biodegradation. In the figure and in Table 2. it is seen that the consume of oils from water is very efficient (80 - 90%). More than extractable oil remnants there are insoluble floating solid material. This is composed of microbe remnants and metabolic products that remind humus. Most of oil mass is removed as gaseous products (carbon dioxide) and water and probably as water soluble products.

### Example 8.

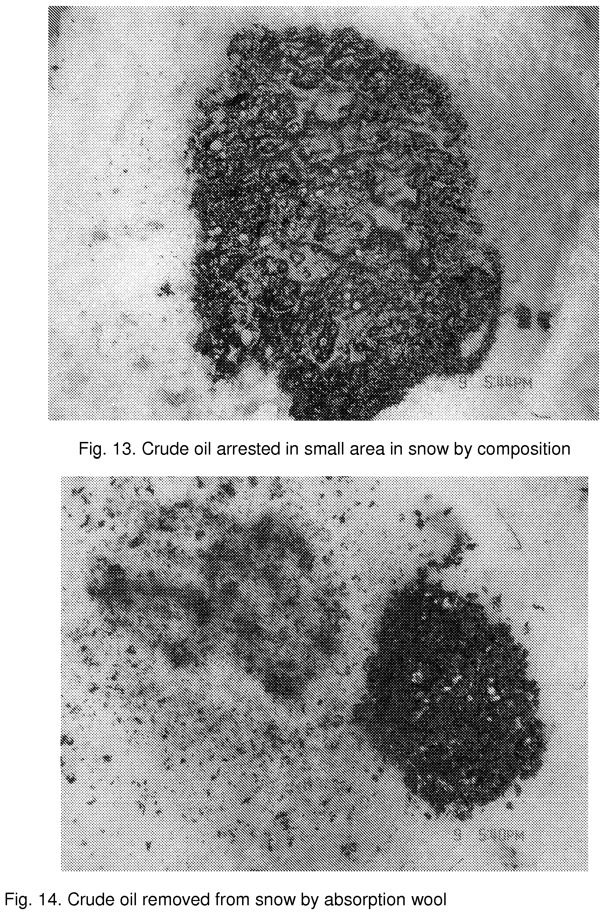

150 grams of Russian crude oil was poured into icy snow. Oil started to spread quickly in wet snow. Two grams of composition was added into oil which made a gel and the spreading was stopped (Fig. 13). The oil was removed by absorption wool mixture of powder and cellulose wool (1 : 3) Fig. 14.

### Example 9.

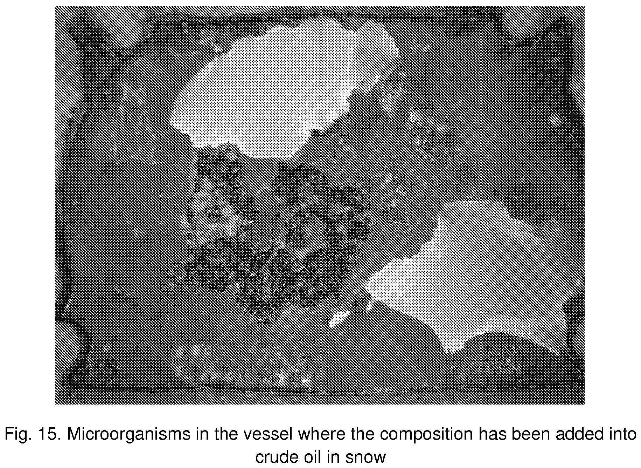

40 litres of icy snow was packed into plastic vessel. 120 grams of Russian crude oil was poured into snow. Two grams of powder arrested the oil and five grams of cellulose wool mixed with powder was added among the oil. The vessel was left outdoors where the temperature was a little over zero of Celsius so that the snow gradually melted. The oil was arrested mainly into wool and after three weeks (when outdoor temperature was over 5°C) there was clear growth of microorganisms in the vessel (Fig. 15)

**Table 2.**

| **Oil** | **Oil, g** | **Oil remnant, g** | **Humus, g** | **Loss of oil (%)** | **Oil remnant and humus, g** | **Loss of total** |
|---|---|---|---|---|---|---|
| | **0 days** | **90 days** | **90 days** | **90 days** | | **mass %** |
| Crude oil + powder | 125,0 | 12,4 | 31,8 | 90,1 | 44,2 | 64,6 |
| Fuel oil + powder | 125,0 | 14,9 | 25,9 | 88,1 | 40,8 | 67,4 |
| Waste lubr. oil + powder | 130,0 | 28,6 | 42,9 | 78,0 | 71,5 | 45,0 |
| Fuel oil+ powder and cellulose wool | 130,0 | 27,7 | 40,3 | 78,7 | 68,0 | 47,7 |
| Crude oil + powder and linen wool | 110,0 | 17,2 | 22,8 | 84,4 | 40,0 | 63,6 |
| Crude oil + powder and cellul. wool | 110,0 | 14,4 | 22,8 | 86,9 | 37,2 | 66,2 |

## Claims

1. A composition for soaking up and biodegradation of oil products and other organic chemicals, **characterised in that** said composition comprises at least:
- a mineral carrier having pores and a vegetable oil or fat and carbonate compounds, which together make oleofobic surface on the mineral particle, and
- nitrogen nutrients and phosphorus nutrients.

2. The composition according to the claim 1, **characterised in that** said mineral carrier material is a surface catalytic material, such as a natural or synthetic silicate mineral

3. The composition according to the claim 1 or 2, **characterised in that** the oleofobic surface has been provided with vegetable oil and/or fat and with carbonate compound(s).

4. The composition according to the claim 1 or 2, **characterised in that** the oleofobic surface has been provided with vegetable oil and/or fat and with carbonate compound(s) and iron.

5. The composition according to any one of the proceeding claims, **characterised in that** said composition further comprises iron and/or manganese.

6. The composition according to any one of the proceeding claims, **characterised in that** the composition further comprises an ingredient that can absorb oil products and/or organic chemicals, such as cellulose fibres or other biodegradable vegetable fibres.

7. A method for manufacturing a composition for soaking and biodegradation of oil products and organic chemicals, **characterised in that** the method comprises at least following steps:
- providing a mineral carrier having pores,
- adding nitrogen nutrients and phosphorus nutrients to said mineral carrier,
- providing oleofobic surface of a vegetable oil or fat and carbonate compounds to said mineral carrier.

8. A method according to claim 7, **characterised in that** said the method additionally comprises the following steps:
adding iron and/or manganese compounds to said mineral carrier.

9. A method according to claim 7 or 8, **characterised in that** oleofobic surface is provided with vegetable oil and/or fat and with carbonate compound(s).

10. A method according to claim 7 or 8, **characterised in that** oleofobic surface is provided with vegetable oil and/or fat and with carbonate compound(s) and iron.

11. A method according to any of claims 7 to 10, **characterised in that** said mineral carrier material is a surface catalytic material, such as a natural or synthetic silicate mineral.

12. A method according to any of claims 7 to 11, **characterised in that** said composition is further provided with an ingredient that can absorb oil products and/or organic chemicals, such as cellulose fibres or other biodegradable vegetable fibres.

13. Use of a composition according to any one of the claims 1 to 6 for soaking up and/or biodegradation of oil products and similar hydrocarbons and other organic chemicals in liquid and/or in solid phase process.

## Patentansprüche

1. Zusammensetzung zum Aufsaugen und biologischen Abbau von Ölprodukten und anderen organischen Chemikalien, **dadurch gekennzeichnet, dass** die Zusammensetzung mindestens Folgendes umfasst:
- einen Mineralträger mit Poren und einem Pflanzenöl oder Fett und Carbonatverbindungen, die zusammen eine oleophobe Oberfläche auf dem Mineralpartikel bilden, und
- Stickstoffnährstoffe und Phosphornährstoffe.

2. Zusammensetzung nach Anspruch 1, **dadurch gekennzeichnet, dass** das Mineralträgermaterial ein oberflächenkatalytisches Material ist, wie beispielsweise ein natürliches oder synthetisches Silikatmineral.

3. Zusammensetzung nach Anspruch 1 oder 2, **dadurch gekennzeichnet, dass** die oleophobe Oberfläche mit Pflanzenöl und/oder Fett und mit Carbonatverbindung(en) versehen wurde.

4. Zusammensetzung nach Anspruch 1 oder 2, **dadurch gekennzeichnet, dass** die oleophobe Oberfläche mit Pflanzenöl und / oder Fett und mit Carbonatverbindung(en) und Eisen versehen wurde.

5. Zusammensetzung nach einem der vorstehenden Ansprüche, **dadurch gekennzeichnet, dass** die Zusammensetzung ferner Eisen und/oder Mangan umfasst.

6. Zusammensetzung nach einem der vorstehenden Ansprüche, **dadurch gekennzeichnet, dass** die Zusammensetzung ferner einen Inhaltsstoff umfasst, der Ölprodukte und/oder organische Chemikalien wie Cellulosefasern oder andere biologisch abbaubare Pflanzenfasern absorbieren kann.

7. Verfahren zur Herstellung einer Zusammensetzung zum Aufsaugen und biologischen Abbau von Ölprodukten und organischen Chemikalien, **dadurch gekennzeichnet, dass** das Verfahren mindestens die folgenden Schritte umfasst:
- Bereitstellen eines Mineralträgers mit Poren,
- Hinzufügen von Stickstoffnährstoffen und Phosphornährstoffen zu dem Mineralträger,
- Bereitstellen einer oleophoben Oberfläche eines Pflanzenöls oder eines Fettes und Carbonatverbindungen zu dem Mineralträger.

8. Verfahren nach Anspruch 7, **dadurch gekennzeichnet, dass** das Verfahren zusätzlich die folgenden Schritte umfasst:
Hinzufügen von Eisen- und/oder Manganverbindungen zu dem Mineralträger.

9. Verfahren nach Anspruch 7 oder 8, **dadurch gekennzeichnet, dass** die oleophobe Oberfläche mit Pflanzenöl und/oder Fett und mit Carbonatverbindung(en) versehen ist.

10. Verfahren nach Anspruch 7 oder 8, **dadurch gekennzeichnet, dass** die oleophobe Oberfläche mit Pflanzenöl und/oder Fett und mit Carbonatverbindung(en) und Eisen versehen ist.

11. Verfahren nach einem der Ansprüche 7 bis 10, **dadurch gekennzeichnet, dass** das Mineralträgermaterial ein oberflächenkatalytisches Material ist, wie beispielsweise ein natürliches oder synthetisches Silikatmineral.

12. Verfahren nach einem der Ansprüche 7 bis 11, **dadurch gekennzeichnet, dass** die Zusammensetzung ferner mit einem Inhaltsstoff versehen ist, der Ölprodukte und/oder organische Chemikalien wie Cellulosefasern oder andere biologisch abbaubare Pflanzenfasern absorbieren kann.

13. Verwendung einer Zusammensetzung nach einem der Ansprüche 1 bis 6 zum Aufsaugen und/oder biologischen Abbau von Ölprodukten und ähnlichen Kohlenwasserstoffen und anderen organischen Chemikalien in Flüssig- und/oder Festphasenverfahren.

## Revendications

1. Composition destinée à absorber et à biodégrader des produits pétroliers et d'autres produits chimiques organiques, **caractérisée en ce que** ladite composition comprend au moins :
- un support minéral ayant des pores et une huile ou une graisse végétale et des composés de carbonate, qui forment ensemble une surface oléophobe sur la particule minérale, et
- des nutriments azotés et des nutriments phosphorés.

2. Composition selon la revendication 1, **caractérisée en ce que** ledit matériau de support minéral est un matériau catalytique de surface, tel qu'un minéral de silicate naturel ou synthétique.

3. Composition selon la revendication 1 ou 2, **caractérisée en ce que** la surface oléophobe a été pourvue d'huile et/ou de graisse végétale et de composé(s) de carbonate.

4. Composition selon la revendication 1 ou 2, **caractérisée en ce que** la surface oléophobe a été pourvue d'huile et/ou de graisse végétale et de composé(s) de carbonate et de fer.

5. Composition selon l'une quelconque des revendications précédentes, **caractérisée en ce que** ladite composition comprend en outre du fer et/ou du manganèse.

6. Composition selon l'une quelconque des revendications précédentes, **caractérisée en ce que** la composition comprend en outre un ingrédient qui peut absorber des produits pétroliers et/ou des produits chimiques organiques, tels que des fibres de cellulose ou d'autres fibres végétales biodégradables.

7. Procédé de fabrication d'une composition destinée à absorber et à biodégrader des produits pétroliers et des produits chimiques organiques, **caractérisé en ce que** le procédé comprend au moins les étapes suivantes :
- la fourniture d'un support minéral ayant des pores,
- l'ajout de nutriments azotés et de nutriments phosphorés audit support minéral,
- la fourniture d'une surface oléophobe d'une huile ou d'une graisse végétale et de composants de carbonate audit support minéral.

8. Procédé selon la revendication 7, **caractérisé en ce que** ledit procédé comprend en outre les étapes suivantes :
l'ajout de composés de fer et/ou de manganèse audit support minéral.

9. Procédé selon la revendication 7 ou 8, **caractérisé en ce que** la surface oléophobe est pourvue d'huile et/ou de graisse végétale et de composé(s) de carbonate.

10. Procédé selon la revendication 7 ou 8, **caractérisé en ce que** la surface oléophobe est pourvue d'huile et/ou de graisse végétale et de composé(s) de carbonate et de fer.

11. Procédé selon l'une quelconque des revendications 7 à 10, **caractérisé en ce que** ledit matériau de support minéral est un matériau catalytique de surface, tel qu'un minéral de silicate naturel ou synthétique.

12. Procédé selon l'une quelconque des revendications 7 à 11, **caractérisé en ce que** ladite composition est en outre pourvue d'un ingrédient qui peut absorber des produits pétroliers et/ou des produits chimiques organiques, tels que des fibres de cellulose ou d'autres fibres végétales biodégradables.

13. Utilisation d'une composition selon l'une quelconque des revendications 1 à 6 destinée à absorber et/ou à biodégrader des produits pétroliers et des hydrocarbures similaires et d'autres produits chimiques organiques dans un procédé en phase liquide et/ou solide.
